# EUROPEAN PATENT SPECIFICATION

(11) **EP 4 473 282 B1**
(45) Date of publication and mention of the grant of the patent: **27.05.2026**
(21) Application number: 23704403.7
(22) Date of filing: 02.02.2023
(51) Int. Cl.: G01N 1/10, A61B 10/00, B01L 3/02, G01N 1/02

(54) **SAMPLE PROCESSING METHODS**
PROBENVERARBEITUNGSVERFAHREN
PROCÉDÉS DE TRAITEMENT D'ÉCHANTILLON

(30) Priority: 02.02.2022 US 202263305866 P; 01.09.2022 US 202263403197 P
(43) Date of publication of application: 11.12.2024
(73) Proprietor: LUMIRADX UK LTD, London SE1 2AQ (GB)
(72) Inventor: BOINARD, Pascal Didier, London Riverside London SE1 2AQ (GB); MCINTOSH, Lesley Anne, London Riverside London SE1 2AQ (GB)
(74) Representative: Marks & Clerk LLP
(86) International application number: PCT/GB2023/050226
(87) International publication number: WO 2023/148489

(56) References cited:
- WO-A1-2021/209228
- CA-A1- 3 164 354
- SG-A- 102020 046 76W
- US-A- 3 077 780
- US-A- 3 191 813
- US-A1- 2007 207 064
- US-A1- 2017 036 205

## Description

### FIELD OF THE INVENTION

The present invention relates to methods for collecting and manipulating liquid samples, as defined in the claims. Also disclosed are related devices.

### BACKGROUND OF THE INVENTION

Liquid samples, e.g., blood, urine, and saliva, may be subjected to diagnostic assays such as to determine the presence of one or more targets present in the sample or to determine a physio-chemical property of the sample. For example, a sample of blood may be analyzed to determine the amount of glycated hemoglobin (HbA1c) present therein. In connection with performing such assays, steps such as sample collection and processing may be performed. For example, in performing diagnostic assay to determine HbA1c in a blood sample, an amount of blood produced from a fingerstick may be collected and subjected the blood to processing such as by lysing red blood cells therein to render the HbA1c accessible to reagents used in the diagnostic assay.

US 2007/207064 A1 relates to a method according to the preamble of claim 1, in which an annular or spiral droplet holder formed of wire is used to hold a droplet in a state of being hung therefrom or being contained therein. A means for moving the droplet holder is added to the droplet holder to enable droplet transfer. To merge two droplets, they are brought into contact. To drip the droplet held by a droplet holder formed of wire, the droplet holder is deformed using an external force. A light path which passes through a droplet is set to enable optical measurement.

US 2017/036205 A1 relates to fluid collection device comprising an elongate container having an opening at one end; a swab that is insertable into the container through said opening; a shaft, the absorbent collector being at or near a distal end of the shaft; and a closure being at or near a proximal end of the shaft. The swab comprises an absorbent collector, for acquiring a fluid sample in use. The closure is adapted to engage with the container and to close the opening of the container with the absorbent collector inside the container.

SG 10202004676WA relates to a swab comprising a rod having a longitudinal axis and a tip. The tip includes a proximal region at a distal end of the rod, a distal region opposite the proximal region, and a plurality of straight blades extending from the proximal region to the distal region, parallel to the longitudinal axis.

### SUMMARY OF THE INVENTION

In embodiments, a method includes contacting a liquid sample with one or more collection apertures of a sample collection annulus to collect a film of the liquid sample within each of the one or more collection apertures. The method may further include inserting the sample collection annulus with the collected liquid sample film(s) into a tubular container through an opening thereof.

In embodiments, the tubular container includes a reagent, e.g., a liquid reagent disposed therein during the step of inserting. In embodiments, the method includes introducing a reagent, e.g., a liquid reagent to the tubular container concurrently with and/or following the step of inserting the sample collection annulus into the tubular container. In embodiments in which the container includes a liquid reagent disposed therein (whether present during the step of inserting or introduced concurrently with and/or following the step of inserting), the step of inserting may include at least partially, e.g., entirely, immersing the sample collection annulus and the collected liquid sample film(s) within the liquid reagent.

In any of the embodiments, the step of contacting may include manipulating, e.g., manually via an operator's fingers, the sample collection annulus with a tubular dispenser secured with respect to the sample collection annulus. The sample collection annulus and the tubular dispenser may be secured with respect to one another, e.g., indirectly by an extending arm. In embodiments, the one or more collection apertures of the sample collection annulus each define a plane and the tubular dispenser defines an internal bore extending along an axis that is generally parallel to the plane of the collection aperture(s).

In any of the embodiments including a liquid reagent, the method may further include dispensing at least some of the liquid and the collected liquid sample from the tubular container through the tubular dispenser.

In any of the embodiments including a liquid reagent, the method may include mixing the liquid reagent and the collected film(s) of liquid sample within the tubular container prior to or concurrently with the step of dispensing.

In any embodiments of the method, the one or more collection apertures may be triangular. In any of the embodiments, the one or more collection apertures of the sample collection annulus having a total area of between about 2 mm² and 10 mm², between about 4 mm² and 8 mm² e.g., an area of about 4 mm², of about 5 mm², about 6 mm², about 7 mm², or about 7.6 mm². In any of the embodiments, the one or more collection apertures of the sample collection annulus have a total volume of between about 4 µl and 12 µl, between about 4 µl and 20 µl, between about 5 µl and 18 µl, between about 7 µl and 16 µl, e.g., about 8 µl, about 10 µl, or about 12 µl or about 13 µl (13 mm³).

In any of the embodiments of the method, the sample collection annulus has a thickness of between about 0.3 mm and 4 mm, between about 0.3 mm and about 2 mm, between about 0.3 mm and about 1 mm, e.g., about 0.5 mm. In any of the embodiments of the method, the sample collection annulus has a maximum thickness and a minimum thickness. The maximum thickness may have any of the aforementioned thickness dimensions and the minimum thickness may be less than about 75%, less than about 65%, less than about 55% or less than about 50% of the maximum thickness.

In any of the embodiments of the method, the sample collection annulus has a height of between about 1 mm and about 3 mm, e.g., between about 1.5 mm and about 2.5 mm, e.g., between about 1.75 mm and about 2.25 mm.

In any of the embodiments of the method, the sample collection annulus has a single collection aperture that defines substantially all, e.g., all, of the area and/or volume of the collection apertures of the sample collection annulus. For example, the sample collection annulus may have a single collection aperture. In any of the embodiments of the method, each of the one or more collection apertures may be open, e.g., free of a grid or mesh extending thereacross.

In any of the embodiments of the method, the method may further include, after the step of inserting the sample collection annulus with the collected liquid sample film(s) into a tubular container through an opening thereof, covering the opening with the sample collection annulus optionally remaining in the tubular container. The step of covering the opening may include covering the opening with a cap having a passage extending therethrough and then dispensing at least some of the collected liquid film(s) and at least some liquid reagent if present from the tubular container through the passage of the cap.

In any of the embodiments of the method, the method may further include, after the step of inserting the sample collection annulus with the collected liquid sample into a tubular container through an opening thereof, covering the opening of the tubular container with a cap having a dispensing passage extending therethrough. The method may further include dispensing at least some of the collected liquid film(s) and at least some liquid reagent if present from the tubular container through the tubular dispenser and through the dispensing passage of the cap.

Further disclosed herein is a system for preparing a liquid sample for an assay including: a sample collection annulus having a collection aperture configured to collect a film of the liquid sample therein; a sample collection arm coupled to the sample collection annulus, the sample collection arm having a top portion and a bottom portion, the top portion detachably secured to the bottom portion, the bottom potion coupled to the sample collection annulus; and a tubular container configured to receive the sample collection annulus and at least the bottom portion through an opening thereof, the tubular container having a chamber therein configured to contain the liquid sample.

The system may further include a dispenser configured to cover the opening of the tubular container, the dispenser having a channel therein that becomes in fluid communication with the chamber when the dispenser covers the opening. The tubular container may include a buffer, e.g., lysis buffer, therein for mixing with the liquid sample. The sample collection annulus may be disposed at an angle with respect to a longitudinal axis of the sample collection arm, e.g., an angle from about 10 degree to about 70 degree, from about 20 degree to about 50 degree, from about 25 degree to about 40 degree, or about 30 degree.

In any embodiment of the use of any annulus disclosed herein, a collected liquid sample disposed within the annulus may be removed therefrom without inserting a tool or member, e.g., a plunger, into or through the annulus. For example, the collected liquid sample may be removed by immersing, either partially or entirely, the annulus into another liquid, such as a buffer or other liquid reagent disclosed herein.

### BRIEF DESCRIPTION OF THE FIGURES

FIGURE (FIG.) 1 is a side view of a sample processing device of the invention, the sample processing device having a hermetically sealed tubular container containing a liquid reagent and a sample manipulation unit;
FIGURE 2 is a side view of the sample manipulation unit of the sample processing device of FIGURE 1, with the side view of FIGURE 2 being taken along a dimension perpendicular to the side view of FIGURE 1; and
FIGURE 3 is a magnified side view of a sample collector of the sample manipulation unit of the sample processing device of FIGURE 1, with the side view of FIGURE 3 being taken along the same dimension as the side view of FIGURE 1;
FIGURE 4 depicts another exemplary sample processing device, having a sample collection annulus, a sample collection arm, a sample collection tube, and a dispenser, according to an embodiment herein;
FIGURES 5A - 5B depict an exemplary side perspective view of the sample collection arm and sample collection annulus of the device from FIGURE 4, according to an embodiment herein;
FIGURES 5C - 5D respectively depict an exemplary top side perspective view and bottom side perspective view of the sample collection arm and sample collection tube of the device from FIGURE 4, according to an embodiment herein; and
FIGURE 6 depicts an exemplary method for preparing a liquid sample for an assay using the device from FIGURE 4, according to an embodiment herein.

### DETAILED DESCRIPTION

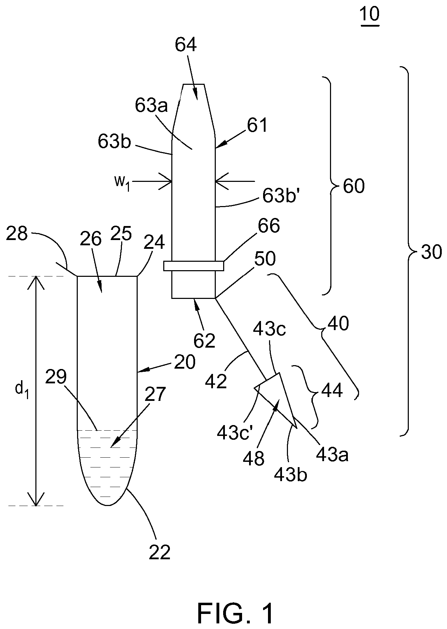

With reference to FIGURE 1, a sample processing device 10 includes a tubular container 20 and a sample manipulation unit 30. Sample manipulation unit 30 includes a sample collection unit 40 and a tubular dispenser 60. Tubular container 20 contains a liquid reagent. An operator uses sample collection unit 40 to collect a liquid sample and introduce the collected liquid sample to tubular container 20. The operator then agitates tubular container 20 to mix the collected liquid sample with the liquid reagent disposed therein. The operator dispenses an amount of the mixture of the liquid reagent and the collected liquid sample from tubular dispenser 60 to a diagnostic device to facilitate determination of one or more targets present in the liquid sample and/or determination of a physicochemical property of the sample such as hematocrit in the case of a liquid sample including whole blood.

Tubular container 20 has a base 22 and an access end 24 with an access opening 26 defining a lip 25. Tubular container 20 has a length d1 of about 30 mm. In an upright operating orientation as shown in FIGURE 1, an amount of a liquid reagent such as a lysis buffer 27 having an upper surface 29 is disposed within base 22 of tubular container 20. Lysis buffer 27 contains reagents configured to lyse red blood cells in a sample of capillary blood while preserving targets of interest therein, e.g., hemoglobin, for analysis, e.g., immunological analysis. The volume of lysis buffer 27 is about 100 µl. As provided to an operator, access opening 26 is sealed with a removable hermetic seal 28, such as a laminate of foil and a polymer. Hermetic seal 28 prevents lysis buffer 27 from leaking or evaporating prior to use. Tubular container 20 is formed of a material suitable for holding biological samples such as glass or a polymer such as a plastic, e.g., polyethylene, polypropylene or polyethylene terephthalate (PET).

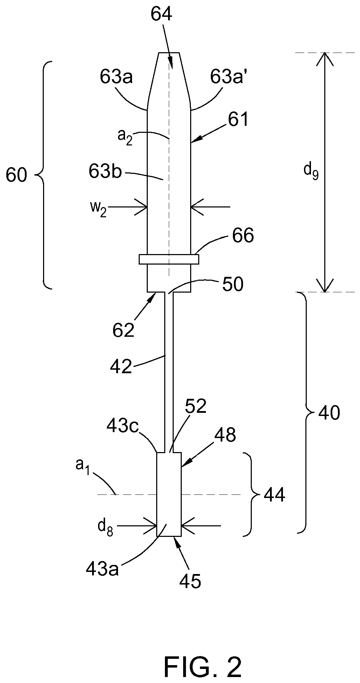

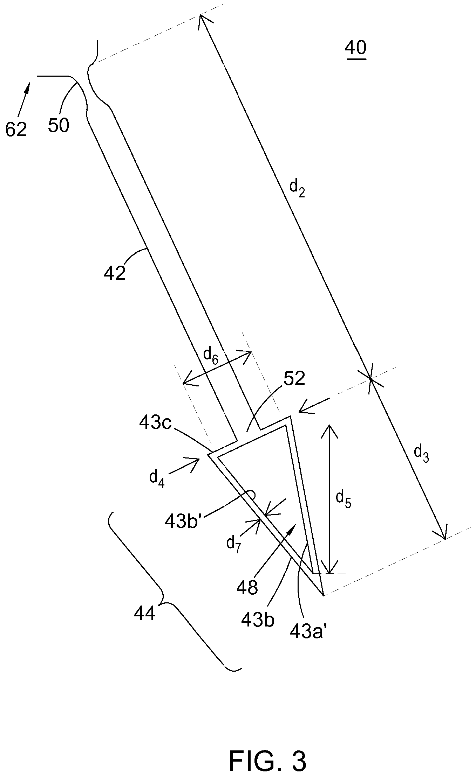

Referring also to FIGURES 2 and 3, sample collection unit 40 and tubular dispenser 60 of sample manipulation unit 30 are integrally secured with respect to one another at an attachment point 50. Proceeding distally from attachment point 50, sample collection unit 40 includes an extending sample collection arm 42 and a sample collection annulus 44. Sample collection arm 42 may be flexible to facilitate inserting sample collection unit 40 into tubular container 20. Sample collection arm 42 and sample collection annulus 44 are integrally secured with respect to one another at an attachment point 52.

In some cases, the sample collection arm 42 and the sample collection annulus 44 are co-linear, such that the sample collection arm and the sample collection annulus extend along the same or substantially the same longitudinal axis. In some cases, the sample collection annulus extends along a longitudinal axis (e.g., annulus axis) that is not co-linear with a longitudinal axis of the sample collection arm (e.g., arm axis). For example, in some cases the annulus axis extends away from the arm axis at an angle from about 0.1 degree to about 90 degree, from about 1 degree to about 75 degree, from about 10 degree to about 50 degree, from about 20 degree to about 40 degree, from about 25 degree to about 35 degree, or about 30 degree. In some embodiments, the transition of the sample collection arm extending along the arm axis to the sample collection annulus extending along the annulus axis occurs at or about the attachment point 52.

Sample collection arm 42 has a length d2 of about 13 mm between attachment points 50,52. Sample collection unit 40 and tubular dispenser 60 are made of a material suitable for manipulating biological samples, e.g., a material having the same or similar composition to the polymer materials of tubular container 20, e.g., polyethylene.

Sample collection annulus 44 has sides 43a, 43b and a base 43c, an outer length d3 of about 5 to 7 mm, and an outer width d4 of about 4 to 5 mm. Each side 43a, 43b and base 43c have a thickness d7 of about 0.4 to 0.6 mm and a height d8 of about 1 to 2 mm, e.g., about 1.75 mm. Sides 43a, 43b meet at a distal terminus 45 of sample collection annulus 44. Sides 43a, 43b define respective inner surfaces 43a', 43b, and base 43c defines an inner surface 43c'. Together, inner surfaces 43a', 43b', 43c' define a sample collection aperture 48 therebetween. Sides 43a, 43b, 43c generally define a plane perpendicular to an axis a1 extending through aperture 28. Each inner surface 43a', 43b' of respective side 43a, 43b has a length d5 of about 4 mm to about 5mm, e.g., about 4.75 mm. Inner surface 43c' of side 43c has a length d6 of about 2.5 to 5 mm, e.g., about 4.2 mm. Sample collection aperture 48 has an area (1/2 × d5 × d6) from about 6 mm² to about 8 mm² and a volume (1/2 × d5 × d6 × d8) from about 11 µl (11 mm³) to about 16 µl (16 mm³), e.g., about 13.3 µl (13.3 mm³). A ratio of the area (in mm²) to the height d8 (in mm) of aperture 48 (area:height) is from about 3 to about 8, e.g., about 4.3. A ratio of the area (in mm²) to the volume (in mm³) of aperture 48 (area:volume) is from about 0.3 to about 0.7, e.g., about 0.6. A ratio of the volume of lysis buffer 27 to the volume of aperture 48 (vol. buffer:vol. aperture) is from about 5 to about 20.

Tubular dispenser 60 includes a tubular wall 61 defining an internal bore extending along an axis a2 between an input opening 62 and a dispensing opening 64. Axis a2 of the bore of tubular wall 61 is generally parallel to the plane defined by the sides of sample collection annulus 44 and is perpendicular to axis a1 extending through aperture 48 of sample collection annulus 44. Tubular wall 61 has an elliptical cross-section along a dimension perpendicular to the longitudinal axis of the internal bore with opposed major surfaces 63a, 63a' and opposed minor surfaces 63b, 63b'. Tubular wall 61 has a constant thickness of about 500 µm and a major external width w1 with a maximum of about 5.8 mm and a minor external width w2 with a maximum of about 2.8 mm. The internal bore of tubular wall 61 has a length d9 of about 20 mm between openings 62,64 and tapers from a maximum internal diameter of about 5.5 mm adjacent input opening 62 to a minimum internal diameter of about 1.8 mm adjacent dispensing opening 64. Tubular member 61 is composed of a flexible polymer so that major surfaces 63a, 63a' can be compressed by an operator's fingers to dispense a sample liquid from dispensing opening 64.

Tubular dispenser 60 includes a flange 66 configured to engage lip 25 of access opening 26 of tubular container 20. In use, inlet opening 62 of tubular wall 61 is inserted into access opening 26 of tubular container 20 until flange 66 engages lip 25. The operator then rotates tubular dispenser 60 with respect to tubular container 20 to secure the two components together. In such operatively secured state, aperture 48 of sample collection annulus 44 is completely immersed within lysis buffer 27 below surface 29 thereof. In such state, a gap, e.g., of several mm, is provided between distal terminus 45 of sample collection annulus 44 and an inner surface of base 22 of tubular container 20 to facilitate complete mixing of the collected sample and lysis buffer 27. Dispensing opening 64 of tubular dispenser 60 may be provided with a cap (not shown) to hermetically seal a collected sample within sample processing device 10.

Sample processing device 10 may be used as follows. An operator who will perform a diagnostic test for HbA1c removes sample processing device 10 from packaging. The operator places tubular container 20 in the upright operating position in a stand (not shown) so that lysis buffer 27 pools in base 22 of tubular container 20. The operator removes hermetic seal 28 from tubular container 20 exposing access opening 26. The operator pricks a finger of a test subject with a lancet to produce a droplet of capillary blood. The operator then touches aperture 48 of sample collection annulus 44 to the capillary blood droplet. An amount of the droplet forms a film of capillary blood having a pair of opposed menisci within aperture 48. The volume of the film of collected capillary blood is determined by the volume of aperture 48 and the shape of the menisci of the film. The shape of the menisci may depend at least in part on the surface energy (e.g., hydrophobicity or hydrophilicity) of inner surfaces 43a', 43b', 43c' of aperture 48 and the nature of the sample, e.g., whether the sample is polar such as an aqueous sample, non-polar such as a non-polar organic solvent, or includes one or more surfactants. For example, in the case of a capillary blood sample and sample collection annulus 44 when formed of a hydrophobic polymer such as untreated polyethylene, the menisci of the film of collected blood are observed to be convex and the volume of the collected blood is observed to be slightly greater than the volume of aperture 48, e.g., between about 10% and 20% greater, e.g., about 10 µl of collected blood as compared to the 8 µl volume of aperture 48. A ratio of the volume of lysis buffer 27 to the volume of blood collected by sample collection annulus 44 and delivered to tubular container 20 (100 µl buffer volume: 10 µl delivered) is about 10.

The operator then inserts sample collection annulus 44 with the collected capillary blood therein into tubular container 20 via access opening 26 thereof. The operator secures flange 66 of tubular dispenser 60 with respect to lip 25 of tubular container 20 with sample collection annulus 44 (and the collected capillary blood within aperture 48) disposed within tubular container 20 along with lysis buffer 27. Aperture 48 of sample collection annulus 44 is completely immersed within lysis buffer 27 below surface 29 thereof.

The operator then agitates sample processing device 10, e.g., by flicking an exterior surface of tubular container 20, to displace the capillary blood film from aperture 48 and mix the lysis buffer and collected capillary blood. During an incubation period, lysis buffer 27 lyses red blood cells present in the capillary blood. The operator then inverts the sample processing device 10 causing the lysis buffer blood mixture (including lysed blood cells) to enter tubular dispenser 60 via inlet opening 62. The operator squeezes major surfaces 63b, 63b' to dispense an amount of the lysis buffer blood mixture from dispensing opening 64 and into the sample application port of a diagnostic device (not shown). An exemplary diagnostic device is any of the microfluidic devices disclosed in International Publication Nr. WO2021146350A2 (the "325 Application"). The operator operates the diagnostic device to determine the amount of HbA1c present in the capillary blood of the test subject. After dispensing the lysis buffer blood mixture, dispensing opening 64 of the tubular dispenser may be sealed, e.g., with a cap (not shown) to preserve the remaining lysis buffer blood mixture and prevent leakage. Alternatively, sample processing device 10 may be disposed of if no further analysis is desired.

Sample processing device 10, tubular container 20, and sample manipulation unit 30, and disclosed methods of operation, are exemplary. Other configurations and/or methods of use may be implemented.

The dimensions of the sample collection aperture 48 may be different from the dimensions disclosed above. For example, in embodiments, the lengths of inner surfaces 43a',43b', and 43c' are each independently at least about 2 mm long, at least about 2.5 mm long, at least about 3 mm, or at least about 4 mm long. In embodiments, the lengths of inner surfaces 43a',43b', and 43c' are each independently about 6.5 mm or less, about 5.5 mm or less, or about 5 mm or less. In embodiments, at least one, e.g., one, of inner surfaces 43a',43b', and 43c' has a shorter length that is between about 50% and 75%, e.g., about 66%, of the length of the longest length of inner surfaces 43a',43b', and 43c'. In embodiments, two of inner surfaces 43a',43b', and 43c' have essentially the same longest length, e.g., about 4.8 mm and the third inner surface has the shorter length, e.g., about 4.2 mm.

In embodiments, the height d8 of the sample collection aperture is at least about 1 mm, at least about 1.5 mm, or at least about 2 mm. In embodiments, the height of the sample collection aperture is about 3 mm or less, about 2.5 mm or less, about 2.25 mm or less, or about 2 mm or less. For example, the height may be between about 1.75 and 2.25 mm. In embodiments, the area of the sample collection aperture is at least about 4 mm², at least about 5 mm², at least about 6 mm², or at least about 7 mm². In embodiments, the area of the sample collection aperture is about 12 mm² or less, about 10 mm² or less, about 9 mm² or less, or about 8 mm² or less. In embodiments, the volume of the sample collection aperture is at least about 8 mm³, at least about 9 mm³, at least about 10 mm³, or at least about 11 mm³. In embodiments, the volume of the sample collection aperture is about 20 mm³ or less, about 18 mm³ or less, about 16 mm³ or less, about 14 mm³ or less, or about 12 mm³ or less. In embodiments, the ratio of the area to the height of the sample collection aperture is between about 2.5 and 6, between about 3 and 5.5, or between about 3.5 and 4.5. In embodiments, the ratio of the area (in mm²) to the volume (in mm³) of the sample collection aperture is between about 0.3 and 0.8, between about 0.4 and 0.7, e.g., between about 0.5 and 0.6.

While sample collection annulus 44 is described as having a single triangular collection aperture 28, alternative sample collection annulus configurations may be implemented. For example, a sample collection annulus may have more than one collection aperture, e.g., 2, 3, 4 or more collection apertures. Whether in a single or multiple collection aperture configuration, each collection aperture may have a shape other than triangular. For example, a collection aperture may be a polygon having a number of sides greater than 3, e.g., 4, 5, 6 or more sides such that the collection aperture is respectively quadrangular, pentagonal, or hexagonal. A collection aperture may be arcuate in shape, e.g., oval, pyriform, circular, or elliptical. A collection aperture may have a shape that combines linear and arcuate internal surfaces, e.g., an oblong collection aperture. A collection aperture may have other shapes such as a vesica piscis or Reuleaux triangle. As another example, while aperture 48 is described as open within a perimeter defined by inner surfaces 63a', 63b', 63c', a collection aperture may include a grid or mesh within a larger perimeter of the sample collection annulus. The grid or mesh may help support and retain a collected liquid sample within the aperture.

While sample manipulation unit 30 is described as including integrally secured sample collection unit 40 and tubular dispenser 60, other configurations may be implemented. For example, a sample collection unit and a tubular dispenser may be mechanically secured, e.g., via a mechanical connection such as a snap connection or press-fit that optionally permits the two components to be separated from one another. As another example, a sample manipulation unit may include a tubular dispenser and a sample collection annulus that are secured to one another without a sample collection arm or other extension. For example, a sample manipulation unit may include a tubular dispenser secured directly to a sample collection annulus, e.g., the tubular dispenser may be secured directly to a side of the sample collection annulus. A sample manipulation unit may include a sample collection unit secured to a tubular dispenser at a location other than a lip of an opening to the tubular dispenser. For example, a sample collection unit may be secured to a support that extends into or across an inlet opening to the tubular dispenser. Alternatively, the sample collection unit and tubular dispenser may be secured with respect to one another via press fitting.

A sample manipulation unit may include a sample collection unit and a tubular dispenser that are separate in that neither is secured with respect to the other during at least a portion of the time that the sample manipulation unit is in use. For example, sample collection unit may be configured so that an operator manipulates the sample collection annulus independently of a tubular dispenser to collect a liquid sample in one or more collection apertures of the sample collection annulus. For example, the operator may manipulate the sample collection annulus via an arm or extension from a side or outer perimeter thereof. The operator may then place the sample collection annulus (optionally including an arm or extension if present) with collected liquid sample into a tubular container. Such container may be sealed with a tubular dispenser, e.g., a cap that includes a dispensing opening to permit a mixture of a liquid reagent (if present) and liquid sample to be dispensed. Such a sample manipulation unit may alternatively include a tubular dispenser that lacks a capping function, such as a pipette. After placing the separate sample collection annulus in a tubular container, optionally including one or more reagents, an operator may use the pipette to remove an amount of a mixture of reagent (if present) and liquid sample.

While sample collection unit 40 is described as including sample collection arm 42 and sample collection annulus 44, other configurations may be implemented. For example, a sample collection annulus may be provided with an arm or other extension that is relatively rigid or inflexible. Such arm or extension may secure the sample collection annulus to a tubular dispenser or may be separate therefrom. As another example, a sample collection annulus may be provided without an arm or other extension (whether or not flexible). In such embodiment, an operator may manipulate the sample collection annulus via a perimeter thereof to collect a liquid sample, as by contacting the collection aperture(s) of the sample collection annulus to the liquid sample, and then placing the sample collection annulus into a tubular container, e.g., a tubular container containing one or more reagents to form a mixture of the reagent(s) and collected liquid sample.

While sample collection unit 40 of sample manipulation unit 30 is described as positioning aperture 48 of sample collection annulus 44 below surface 29 of lysis buffer, other configurations may be implemented. For example, a sample processing device may be configured to immerse the one or more annular collection apertures of a sample collection annulus only partially within a liquid reagent present in a tubular container. A sample processing device may be configured to position a distal terminus of sample collection annulus adjacent to and above a surface of a liquid reagent within a tubular container. As another example, a sample collection annulus may be configured to position the one or more (e.g., all) collection apertures thereof sufficiently far above the surface of a liquid reagent that the one or more collection apertures do not become immersed within the liquid reagent even with gentle agitation of the tubular container. Such sample processing device may be inverted to immerse the one or more collection apertures and the liquid reagent.

Tubular container 20 is described as containing a liquid reagent, e.g., lysis buffer 27. Other configurations are possible. For example, tubular container may contain no reagents at all. A tubular container may contain only one or more reagents in a dried state therein, e.g., an anticoagulant such as lithium heparin. Such a configuration may be used to, e.g., preserve a sample collected within a collection aperture(s) of a sample collection annulus for subsequent analysis. As another example, a tubular container may include one or more liquid reagents other than, or in addition to, a lysis buffer. Exemplary liquid reagents include water, organic solvent(s), or combination thereof. Other examples of liquid reagents include saline, blood coagulants or anticoagulants, and buffers lacking a lysing capability. Such tubular container, e.g., with no reagents, only dried reagents, or such liquid reagents, may be sealed with a cap to preserve the collected sample.

While tubular container 20 is described as having a volume of about 100 µl of liquid reagent, e.g., lysis buffer 27, other amounts or volumes may be implemented. For example, a tubular container may container a volume of liquid reagent(s) of at least about 5 µl, at least about 10 µl, at least about 25 µl, at least about 50 µl, or at least about 75 µl. A tubular container may contain a volume of liquid reagent(s) of about 300 µl or less, about 250 µl or less, about 200 µl or less, or about 175 µl or less.

While a ratio of the volume of liquid reagent in tubular container 20 to the volume of aperture 48 (vol. buffer:vol. aperture) is described to be about 12.5, other ratios may be implemented. For example, a ratio of the volume of liquid reagent(s) contained in a tubular container to the total volume of one or more apertures of a sample collection annulus may be about 5 or more, about 7.5 or more, or about 10 or more. The ratio of the volume of liquid reagent(s) contained in a tubular container to the total volume of one or more apertures of a sample collection annulus may be about 25 or less, about 20 or less, about 17.5 or less, or about 15 or less.

While a ratio of the volume of liquid reagent within tubular container 20 to the volume of blood collected by sample collection annulus 44 and delivered to tubular container 20 (100 µl buffer volume: 10 µl delivered) is described to be about 10, other ratios may be implemented. For example, a ratio of the volume of liquid reagent(s) contained in a tubular container to the total volume of liquid sample collected and delivered to the tubular container using a sample collection annulus may be about 5 or more, about 7.5 or more, or about 10 or more. The ratio of the volume of liquid reagent(s) contained in a tubular container to the total volume of liquid sample collected and delivered to the tubular container using a sample collection annulus may be about 25 or less, about 20 or less, about 17.5 or less, or about 15 or less.

Sample processing device 10 is described as configured for collecting and processing a capillary blood sample in preparation for a determination HbA1c. A sample processing device and/or sample collection annulus may be used to collect and optionally process other liquid samples including venous blood, urine, saliva, sputum, spinal fluid, and nasopharyngeal samples within one or more apertures of the sample collection annulus. A sample processing device and/or sample collection annulus may be used to collect non-biological samples such as water or other environmental samples. A sample processing device and/or sample collection annulus may be used to collect and process samples in connection with determinations other than HbA1c and/or determinations other than immunological. For example, a sample liquid collected using a sample collection annulus may be analyzed to determine the presence and/or amount of one or more pathogens, one or more inorganic targets such as lactate, or one or more physiochemical properties of a sample such as hematocrit of a blood sample. A sample liquid collected using sample collection annulus may be analyzed with any type of assay or diagnostic technique such as electrochemical, colorimetric or other assay or diagnostic technique indicative of the presence and/or amount of a target or physiochemical property of interest. A sample processing device and/or sample collection annulus may be used to collect and process samples in connection with uses other than performing determinations of targets or properties of samples. For example, a sample processing device may be used to collect samples and dispense the sample or combinations of the sample and reagents for purposes such as drying or preservation.

Sample processing device 10 is described as processing a blood sample by lysing red blood cells therein to release a target, e.g., HbA1c, therein for a diagnostic determination. A sample processing device may perform other steps in the alternative to or in combination with such lysing. For example, processing may include or essentially consist of collecting a liquid sample using a sample collection annulus thereby obtaining a liquid sample suitable for drying or direct introduction to a diagnostic device for determination of the presence and/or amount of a target(s) within the sample or a property of the sample (e.g., hematocrit). Processing may include or consist essentially mixing the collected sample with a liquid reagent that dilutes and optionally preserves components (e.g., targets) within the sample without lysis of cells therein. Processing may include or consist essentially of mixing the collected sample with a reagent including a binding agent to a target(s) therein, e.g., an antibody or other reagent that specifically binds a target(s) with the sample. Processing may include or consist essentially of subjecting the collected sample, whether or not combined with one or more reagents, to centrifugation.

While an operator is described as dispensing a reagent sample mixture from sample processing device 10 by squeezing wall 61 of tubular dispenser 60, other methods may be implemented. For example, a tubular container may be formed of a flexible material permitting a user to dispense a mixture therefrom by squeezing the tubular container as an alternative or in combination with squeezing a wall of a tubular dispenser.

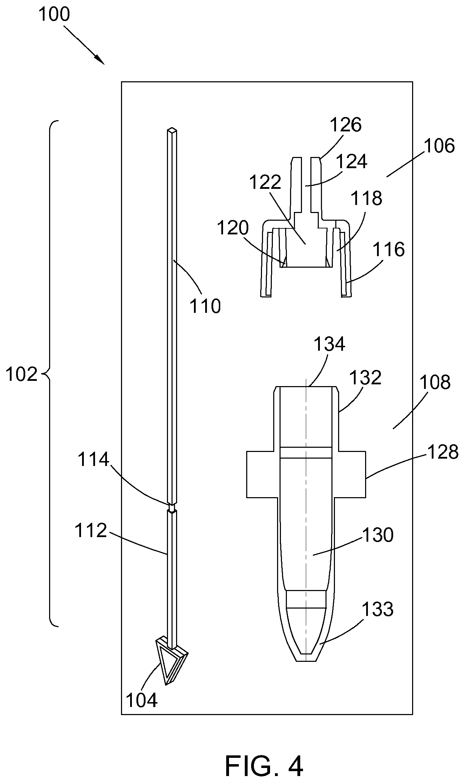

FIG. 4 depicts an alternate embodiment for a sample processing system 100, including a sample collection unit, a tubular container (sample collection tube) 108, and a dispenser 106. The sample collection unit may include a sample collection arm 102 and a sample collection annulus 104. In some cases, the sample collection annulus 104 comprises the features, dimensions, and/or other shapes as described for any other sample collection annulus described herein (e.g., sample collection annulus 44 or variations as described herein).

Sample processing unit 100 comprises a sample collection tube 108 configured to receive the sample collection annulus 104 and a sample collected therein. As described herein, the sample collection tube 104 may include a liquid reagent such as a buffer, e.g., a lysis buffer, therein, for mixing with the sample. The sample collection tube 108 may include a chamber 130 therein for receiving the buffer and the sample through an opening 134. The sample collection tube 108 may include a protrusion configured 128 to mate with a portion of the dispenser 106. The dispenser 106 may include an outer cover having an internal groove 118 configured to mate with a wall 132 defining a body on the sample collection tube 108, thereby securing the dispenser 106 to the sample collection tube 108. The dispenser 106 may include a channel 124 therein to dispense the mixture of the sample and the lysis buffer (e.g. dispense onto an assay device).

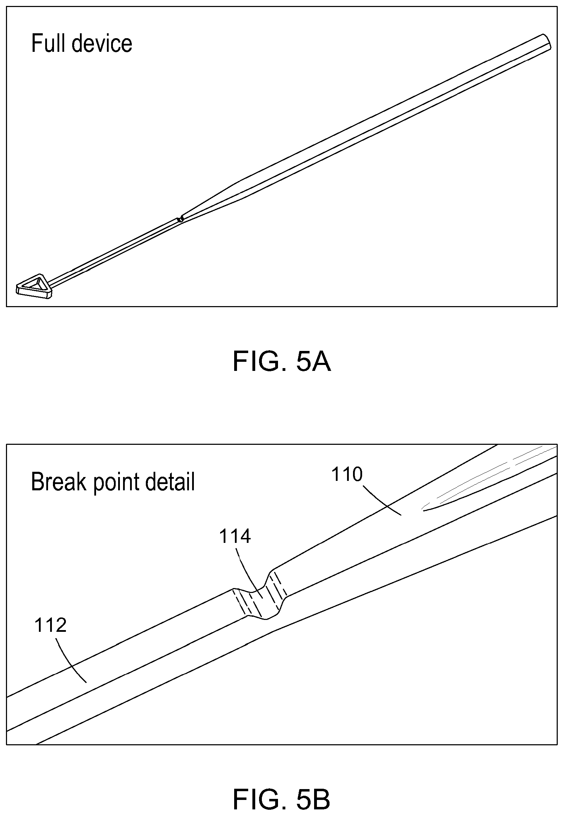

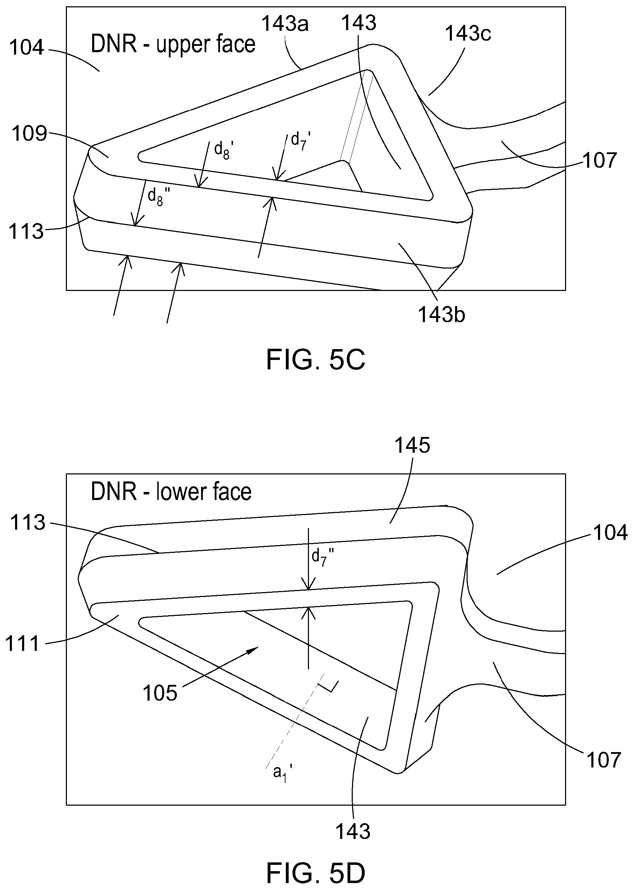

FIGS. 5A-5D show exemplary depictions of a sample collection annulus 104. The sample annulus 104 includes an aperture 105 therein, configured to receive, e.g., collect, a sample (as described herein). Sample collection annulus 104 is defined by sides 143a, 143b, 143c collectively defining an upper surface 109, a lower surface 111, an interior surface 143, and an outer surface 145. Upper surface 109 may alternatively be referred to herein as an upper face and lower surface 111 may alternatively be referred to as a lower face of annulus 104. Sample collection annulus 104 generally defines a plane perpendicular to an axis a1' extending through aperture 105 and a height d8' parallel to axis a1' between upper and lower surfaces 109,111. Height d8' is between about 1 mm and 2.5 mm, e.g., about 1.75 mm. Similar to the sample collection annulus from the device 10, the respective inner surfaces 143 of sides 143a, 143b, 143c define sample collection aperture 105 therebetween.

Side walls 143a, 143b, 143c have a constant thickness d7' in a plane perpendicular to axis a1' between upper surface 109 and a mid-point 113 of the side walls. Proceeding from mid-point 113 to lower surface 111, the thickness of side walls 143a, 143b, 143c tapers from thickness d7' to a reduced thickness d7" at lower surface 111. Thickness d7' is between about 0.3 mm and 0.8 mm, e.g., about 0.5 mm. Alternatively, inner and outer surfaces 143,145 may intersect at lower surface 111. Thickness d7'' is between about 0.05 mm and 0.4 mm, e.g., about 0.25 mm. A ratio of the thickness d7' between mid-point 113 and upper surface 109 to thickness d7'' at lower surface 111 is typically between about 1.5 and about 10, e.g., about 2. The interior surface 143 of each side wall 143a, 143b, 143c is generally planar and parallel with axis a1'. The outer surface 143 of each side wall 143a, 143b, 143c is parallel to the inner surface of each sidewall between upper surface 109 and mid-point 113. The outer surface 145 of each side wall 143a, 143b, 143c tapers inward between mid-point 113 and lower surface 111 by an amount corresponding to the reduced thickness between mid-point 113 and lower surface 111.

In some embodiments, the thickness of side walls 143a, 143b, 143c is constant proceeding from lower surface 111 to mid-point 113, e.g., with a thickness corresponding to thickness d7' and then tapers proceeding toward upper surface 109, e.g., with a thickness corresponding to thickness d7''. In other embodiments, the thickness of side walls 143a, 143b, 143c tapers proceeding from mid-point 113 proceeding toward both upper and lower surfaces 109,111, e.g., from a thickness d7' to a thickness d7''. The tapered configuration of the thickness of side walls 143a, 143b, 143c can help improve sample collection when annulus 104 is inserted through the surface of a sample liquid to collect a volume of the sample liquid within the annulus. For example, the tapered configuration may help pierce the surface of the liquid so that the liquid collects within the annulus. In the absence of the tapered configuration, the surface tension of the liquid may inhibit the annulus from readily passing through the liquid surface.

The sample collection annulus 104 may be include any dimension, feature, and/or shape as described herein. For example, the sample collection annulus may have an outer length analogous to length d3 of annulus 44 of about 5 to 7 mm, e.g., about 5.8 mm, and an outer width analogous to width d4 of annulus 44 of about 3.5 mm to 6 mm, e.g., about 4.2 mm. Inner surfaces of sides 143a, 143b may have a length analogous to length d5 of annulus 44 of about 4 mm to 6 mm, e.g., about 4.8 mm. The inner surface of side 143c may have a length analogous to length d4 of annulus 44 of about 3.5 mm to 5.5 mm, e.g., about 4.2 mm. Alternatively, the lengths of the inner surface of sides 143c may be the same as for the inner surfaces of sides 143a,143b. Sample collection aperture 105 has an area between the inner surfaces of side walls 143a, 143b, 143c of about 6 mm² to about 9 mm², e.g., about 7.6 mm². Sample collection aperture 105 has a volume between the inner surfaces of side walls 143a, 143b, 143c and upper and lower surfaces 109,111 of from about 11 µl (11 mm³) to about 16 µl (16 mm³), e.g., about 13.3 µl (13.3 mm³). A ratio of the area (in mm²) to the height d8 (in mm) of aperture 105 (area:height) is from about 2.5 to about 8, e.g., from about 3 to about 7, e.g., about 4.3. A ratio of the area (in mm²) to the volume (in mm³) of aperture 105 (area:volume) is from about 0.3 to about 0.8, e.g., about 0.6. A ratio of the volume of lysis buffer 27 to the volume of aperture 105 (vol. buffer:vol. aperture) is from about 5 to about 20.

The sample collection arm (102) may be attached to the sample collection annulus (104) at an attachment point 107. As described herein, in some cases, the sample collection arm 102 and the sample collection annulus 104 are co-linear, such that the sample collection arm and the sample collection annulus extend along the same or substantially the same longitudinal axis. In some cases, the sample collection annulus 104 extends along a longitudinal axis (e.g., annulus axis parallel to the plane of collection aperture 105) that is not co-linear with a longitudinal axis of the sample collection arm 102 (e.g., arm axis). For example, in some cases the annulus axis extends away from the arm axis at an angle from about 0.1 degree to about 90 degree, from about 1 degree to about 75 degree, from about 10 degree to about 50 degree, from about 20 degree to about 40 degree, from about 25 degree to about 35 degree, or about 30 degree. In some embodiments, the transition of the sample collection arm extending along the arm axis to the sample collection annulus extending along the annulus axis occurs at or about the attachment point 107.

With reference to FIGs. 5A-5B, the sample collection arm 102 may have a top portion 110 and a bottom portion 112 attached together at a break point location 114. The bottom portion 112 may be configured to be attached to the sample collection annulus 104 (e.g., via attachment point 107). The top portion 110 may be configured to be separated and removed from the bottom portion 114. In some cases, the break point 114 comprises a frangible connection.

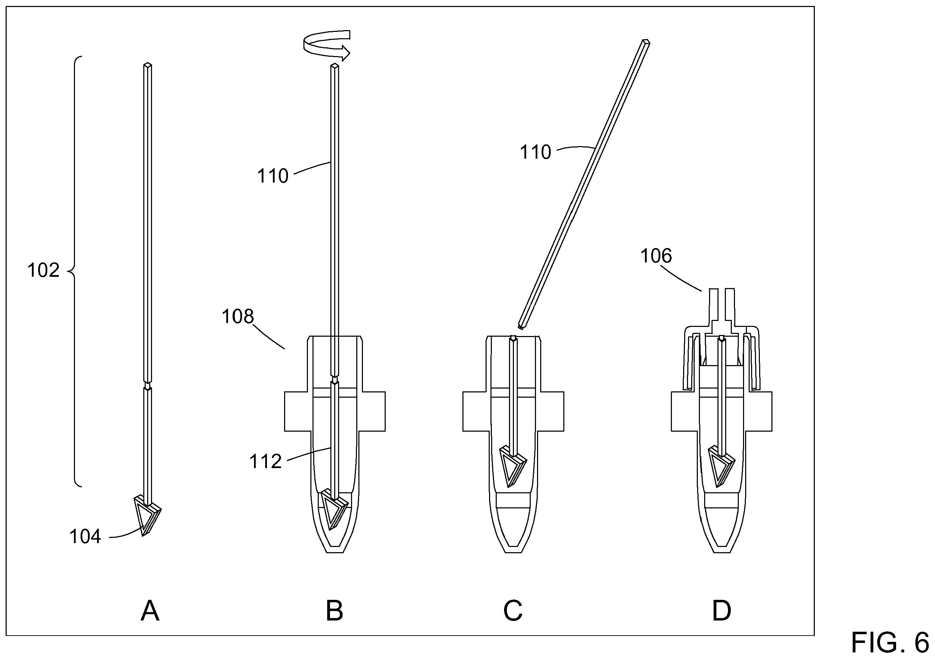

FIG. 6 depicts an exemplary method for processing a sample for an assay. Using the sample collection unit, a liquid sample may first be obtained within the sample collection annulus aperture 105 of the sample collection annulus 104 (A). The sample may be obtained by contacting annulus aperture 105 to blood present on a subject's finger, e.g., blood obtained from a fingerstick. Alternatively, the sample may be obtained by contacting annulus aperture to a liquid sample, e.g., blood, a blood-containing liquid sample, disposed within a container, e.g., an EDTA tube, liquid control container, or other suitable container. For example, an operator may insert annulus 104 into the sample through the surface thereof. As discussed above, the tapered thickness of the sidewalls of annulus 104 may facilitate piercing the surface of the liquid so that the liquid enters collection aperture 105. Sample is retained by surface tension within annulus aperture 105. In the case of a blood sample, e.g., capillary or venous blood, and sample collection annulus 104 when formed of a hydrophobic polymer such as untreated polyethylene, the menisci of the film of collected blood are observed to be convex and the volume of the collected blood is observed to be slightly greater than the volume of aperture 48, e.g., between about 10% and 20% greater, e.g., about 12.5% greater, e.g., about 15 µl of collected blood as compared to the 13.3 µl volume of aperture 48.

After obtaining the sample, the sample is mixed with the lysis buffer disposed within the sample collection tube 108 (B). For example, the sample collection arm 102 may be rotated to swirl the sample collection annulus 104 within the sample collection tube 108. After mixing the sample with the lysis buffer, the top portion 110 of the sample collection arm may be broken off, thereby leaving the bottom portion 112 and the sample collection annulus within the sample collection tube 108 (C). The dispenser 106 is then coupled to the sample collection tube 108, wherein the sample collection tube 108 may be inverted so as to dispense the mixture of the sample and the lysis buffer onto an assay device (e.g., a microfluidic strip) (D).

Sample collection annulus 104 may incorporate any feature, dimension, and/or shape and be used for any sample and/or liquid reagents as disclosed herein for any other sample collection annulus, e.g., annulus 44. Samples collected using annulus 104 may be subjected to any processing, assay and/or diagnostic technique as disclosed herein for any other sample collection annulus, e.g., annulus 44. The sample collection tube 108 and the dispenser 106 may incorporate any feature, dimension, and/or shape from any other sample collection tube and dispenser described herein.

While various specific embodiments have been illustrated and described, the above specification is not restrictive. It will be appreciated that various changes can be made without departing from the scope of the present disclosure(s). Many variations will become apparent to those skilled in the art upon review of this specification.

## Claims

1. A method, comprising:
contacting a liquid sample with a collection aperture (48) of a sample collection annulus (44) to collect a film of the liquid sample within the collection aperture (48); and
inserting the sample collection annulus (44) with the collected film of the liquid sample into a tubular container (20) through an opening (26) thereof.

2. The method of claim 1, wherein the tubular container (20) comprises a liquid disposed therein, wherein the step of inserting comprises at least partially immersing the sample collection annulus (44) and the collected film of the liquid sample within the liquid.

3. The method of claim 1 or 2, wherein the contacting comprises manipulating the sample collection annulus (44) with a tubular dispenser (60) secured with respect to the sample collection annulus (44), wherein the sample collection annulus (44) and the tubular dispenser (60) are indirectly secured with respect to one another by an extending arm.

4. The method of claim 3, wherein the collection aperture (48) of the sample collection annulus (44) defines a plane and the tubular dispenser (60) defines an internal bore extending along an axis that is generally parallel to the plane of the collection aperture (48), further comprising dispensing at least some of the liquid and the collected film of the liquid sample from the tubular container (20) through the tubular dispenser (60).

5. The method of claim 4, wherein the method comprises mixing the liquid and the collected film of the liquid sample within the tubular container (20) prior to or concurrently with the step of dispensing.

6. The method of claim 1 or 2, wherein the contacting comprises manipulating the sample collection annulus (44) with a sample collection arm (42) secured thereto, wherein the sample collection annulus (44) is disposed at an angle with respect to a longitudinal axis of the sample collection arm (42).

7. The method of claim 6, wherein the sample collection arm (42) comprises a top portion (110) and a bottom portion (112), the top portion (110) being separable from the bottom portion (112), the bottom portion (112) being secured to the sample collection annulus (44), wherein the top portion (110) is detachably secured to the bottom portion (112) via a frangible connection (114).

8. The method of any preceding claim, wherein the collection aperture (48) is triangular.

9. The method of any preceding claim, wherein the collection aperture (48) has an area of between about 2 mm² and 10 mm², e.g., an area of about 4 mm², an area of about 5 mm², an area of about 6 mm², an area of about 7 mm², or an area of about 8 mm².

10. The method of any preceding claim, wherein the collection aperture (48) has a volume of between about 4 µl and 20 µl, between about 5 µl and 18 µl, between about 7 µl and 16 µl, e.g., about 8 µl, about 10 µl, or about 12 µl.

11. The method of any preceding claim, further comprising, after the step of inserting the sample collection annulus (44) with the collected film of the liquid sample into a tubular container (20) through an opening (26) thereof, covering the opening (26), wherein the sample collection annulus (44) optionally remains in the tubular container (20).

12. The method of claim 7, wherein the step of covering the opening (26) comprises covering the opening (26) with a cap having a passage extending therethrough and then dispensing at least some of the collected film of the liquid sample from the tubular container (20) through the passage of the cap.

13. The method of any of claims 3-5, further comprising, after the step of inserting the sample collection annulus (44) with the collected film of the liquid sample into a tubular container (20) through an opening (26) thereof, covering the opening (26) of the tubular container (20) with a cap having a dispensing passage extending therethrough.

14. The method of claim 9, further comprising dispensing at least some of the liquid within the tubular container (20) and the collected film of the liquid sample from the tubular container (20) through the tubular dispenser (60) and through the dispensing passage of the cap.

## Patentansprüche

1. Verfahren, umfassend:
Kontaktieren einer Flüssigkeitsprobe mit einer Sammelöffnung (48) eines Probensammelrings (44), um einen Film der Flüssigkeitsprobe innerhalb der Sammelöffnung (48) zu sammeln; und
Einführen des Probensammelrings (44) mit dem gesammelten Film der Flüssigkeitsprobe in einen rohrförmigen Behälter (20) durch eine Aussparung (26) davon.

2. Verfahren nach Anspruch 1, wobei der rohrförmige Behälter (20) eine darin angeordnete Flüssigkeit umfasst, wobei der Schritt zum Einführen mindestens teilweise Eintauchen des Probensammelrings (44) und des gesammelten Films der Flüssigkeitsprobe innerhalb der Flüssigkeit umfasst.

3. Verfahren nach Anspruch 1 oder 2, wobei das Kontaktieren Manipulieren des Probensammelrings (44) mit einem rohrförmigen Spender (60) umfasst, der in Bezug auf den Probensammelring (44) befestigt ist, wobei der Probensammelring (44) und der rohrförmige Spender (60) indirekt durch einen ausziehbaren Arm aneinander befestigt sind.

4. Verfahren nach Anspruch 3, wobei die Sammelöffnung (48) des Probensammelrings (44) eine Ebene definiert und der rohrförmige Spender (60) eine Innenbohrung definiert, die sich entlang einer Achse erstreckt, die im Allgemeinen parallel zur Ebene der Sammelöffnung (48) ist, weiter umfassend Abgeben mindestens eines Teils der Flüssigkeit und des gesammelten Films der Flüssigkeitsprobe aus dem rohrförmigen Behälter (20) durch den rohrförmigen Spender (60).

5. Verfahren nach Anspruch 4, wobei das Verfahren Mischen der Flüssigkeit und des gesammelten Films der Flüssigkeitsprobe innerhalb des rohrförmigen Behälters (20) vor oder gleichzeitig mit dem Abgabeschritt umfasst.

6. Verfahren nach Anspruch 1 oder 2, wobei das Kontaktieren Manipulieren des Probensammelrings (44) mit einem daran befestigten Probensammelarm (42) umfasst, wobei der Probensammelring (44) in einem Winkel in Bezug zu einer Längsachse des Probensammelarms (42) angeordnet ist.

7. Verfahren nach Anspruch 6, wobei der Probensammelarm (42) einen oberen Abschnitt (110) und einen unteren Abschnitt (112) umfasst, wobei der obere Abschnitt (110) vom unteren Abschnitt (112) trennbar ist, der untere Abschnitt (112) am Probensammelring (44) befestigt ist, wobei der obere Abschnitt (110) über eine Sollbruchverbindung (114) lösbar am unteren Abschnitt (112) befestigt ist.

8. Verfahren nach einem vorstehenden Anspruch, wobei die Sammelöffnung (48) dreieckig ist.

9. Verfahren nach einem vorstehenden Anspruch, wobei die Sammelöffnung (48) eine Fläche zwischen etwa 2 mm² und 10 mm², z. B. eine Fläche von etwa 4 mm², eine Fläche von etwa 5 mm², eine Fläche von etwa 6 mm², eine Fläche von etwa 7 mm² oder eine Fläche von etwa 8 mm² aufweist.

10. Verfahren nach einem vorstehenden Anspruch, wobei die Sammelöffnung (48) ein Volumen zwischen etwa 4 µl und 20 µl, zwischen etwa 5 µl und 18 µl, zwischen etwa 7 µl und 16 µl, z. B. etwa 8 µl, etwa 10 µl oder etwa 12 µl aufweist.

11. Verfahren nach einem vorstehenden Anspruch, das weiter nach dem Schritt zum Einführen des Probensammelrings (44) mit dem gesammelten Film der Flüssigkeitsprobe in einen rohrförmigen Behälter (20) durch eine Aussparung (26) davon Abdecken der Aussparung (26) umfasst, wobei der Probensammelring (44) optional im rohrförmigen Behälter (20) verbleibt.

12. Verfahren nach Anspruch 7, wobei der Schritt zum Abdecken der Aussparung (26) Abdecken der Aussparung (26) mit einer Kappe umfasst, die einen Durchgang aufweist, der sich dort hindurch erstreckt und danach Abgeben von mindestens einem Teil des gesammelten Films der Flüssigkeitsprobe aus dem rohrförmigen Behälter (20) durch den Durchgang der Kappe.

13. Verfahren nach einem der Ansprüche 3-5, das weiter nach dem Schritt zum Einführen des Probensammelrings (44) mit dem gesammelten Film der Flüssigkeitsprobe in einen rohrförmigen Behälter (20) durch eine Aussparung (26) davon Abdecken der Aussparung (26) des rohrförmigen Behälters (20) mit einer Kappe, die einen sich dort hindurch erstreckenden Abgabedurchgang aufweist.

14. Verfahren nach Anspruch 9, weiter umfassend Abgeben von mindestens einem Teil der Flüssigkeit innerhalb des rohrförmigen Behälters (20) und des gesammelten Films der Flüssigkeitsprobe aus dem rohrförmigen Behälter (20) durch den rohrförmigen Spender (60) und durch den Abgabedurchgang der Kappe.

## Revendications

1. Procédé, comprenant :
la mise en contact d'un échantillon liquide avec une ouverture de collecte (48) d'un anneau de collecte d'échantillon (44) pour recueillir un film de l'échantillon liquide à l'intérieur de l'ouverture de collecte (48) ; et
l'insertion de l'anneau de collecte d'échantillon (44) avec le film collecté de l'échantillon liquide dans un récipient tubulaire (20) à travers une ouverture (26) de celui-ci.

2. Procédé selon la revendication 1, dans lequel le récipient tubulaire (20) comprend un liquide disposé à l'intérieur, dans lequel l'étape d'insertion comprend l'immersion au moins partielle de l'anneau de collecte d'échantillon (44) et du film collecté de l'échantillon liquide dans le liquide.

3. Procédé selon la revendication 1 ou 2, dans lequel la mise en contact comprend la manipulation de l'anneau de collecte d'échantillon (44) avec un distributeur tubulaire (60) fixé par rapport à l'anneau de collecte d'échantillon (44), dans lequel l'anneau de collecte d'échantillon (44) et le distributeur tubulaire (60) sont indirectement fixés l'un par rapport à l'autre par un bras extensible.

4. Procédé selon la revendication 3, dans lequel l'ouverture de collecte (48) de l'anneau de collecte d'échantillon (44) définit un plan et le distributeur tubulaire (60) définit un alésage interne s'étendant le long d'un axe généralement parallèle au plan de l'ouverture de collecte (48), comprenant en outre la distribution d'au moins une partie du liquide et du film collecté de l'échantillon liquide du récipient tubulaire (20) à travers le distributeur tubulaire (60).

5. Procédé selon la revendication 4, dans lequel le procédé comprend le mélange du liquide et du film collecté de l'échantillon liquide dans le récipient tubulaire (20) avant ou en même temps que l'étape de distribution.

6. Procédé selon la revendication 1 ou 2, dans lequel la mise en contact comprend la manipulation de l'anneau de collecte d'échantillon (44) avec un bras de collecte d'échantillon (42) fixé à celui-ci, dans lequel l'anneau de collecte d'échantillon (44) est disposé à un angle par rapport à un axe longitudinal du bras de collecte d'échantillon (42).

7. Procédé selon la revendication 6, dans lequel le bras de collecte d'échantillon (42) comprend une partie supérieure (110) et une partie inférieure (112), la partie supérieure (110) étant séparable de la partie inférieure (112), la partie inférieure (112) étant fixée à l'anneau de collecte d'échantillon (44), dans lequel la partie supérieure (110) est fixée de manière détachable à la partie inférieure (112) via une connexion fragile (114).

8. Procédé selon une quelconque revendication précédente, dans lequel l'ouverture de collecte (48) est triangulaire.

9. Procédé selon une quelconque revendication précédente, dans lequel l'ouverture de collecte (48) présente une surface comprise entre environ 2 mm² et 10 mm², par exemple une surface d'environ 4 mm², une surface d'environ 5 mm², une surface d'environ 6 mm², une surface d'environ 7 mm², ou une surface d'environ 8 mm².

10. Procédé selon une quelconque revendication précédente, dans lequel l'ouverture de collecte (48) présente un volume compris entre environ 4 µl et 20 µl, entre environ 5 µl et 18 µl, entre environ 7 µl et 16 µl, par exemple environ 8 µl, environ 10 µl ou environ 12 µl.

11. Procédé selon une quelconque revendication précédente, comprenant en outre, après l'étape d'insertion de l'anneau de collecte d'échantillon (44) avec le film collecté de l'échantillon liquide dans un récipient tubulaire (20) à travers une ouverture (26) de celui-ci, la couverture de l'ouverture (26), dans lequel l'anneau de collecte d'échantillon (44) reste facultativement dans le récipient tubulaire (20).

12. Procédé selon la revendication 7, dans lequel l'étape de couverture de l'ouverture (26) comprend la couverture de l'ouverture (26) avec un bouchon présentant un passage s'étendant à travers celui-ci, puis la distribution d'au moins une partie du film collecté de l'échantillon liquide provenant du récipient tubulaire (20) à travers le passage du bouchon.

13. Procédé selon l'une quelconque des revendications 3-5, comprenant en outre, après l'étape d'insertion de l'anneau de collecte d'échantillon (44) avec le film collecté de l'échantillon liquide dans un récipient tubulaire (20) à travers une ouverture (26) de celui-ci, la couverture de l'ouverture (26) du récipient tubulaire (20) avec un bouchon présentant un passage de distribution s'étendant à travers celui-ci.

14. Procédé selon la revendication 9, comprenant en outre la distribution d'au moins une partie du liquide contenu dans le récipient tubulaire (20) et du film collecté de l'échantillon de liquide du récipient tubulaire (20) à travers le distributeur tubulaire (60) et à travers le passage de distribution du bouchon.
